# EUROPÄISCHE PATENTSCHRIFT

(11) **EP 1 470 829 B1**
(45) Veröffentlichungstag und Bekanntmachung des Hinweises auf die Patenterteilung: **14.11.2007**
(21) Anmeldenummer: 04007897.4
(22) Anmeldetag: 01.04.2004
(51) Int. Cl.: A61L 27/56

(54) **Peröser Körper mit antibiotischer Beschichtung, Verfahren zur Herstellung sowie Verwendung**
Porous article having an antibiotic coating, method of manufacture and use
Article poreux ayant un revêtement antibiotique, méthode de fabrication et utilisation

(30) Priorität: 25.04.2003 DE 10318991
(43) Veröffentlichungstag der Anmeldung: 27.10.2004
(73) Patentinhaber: Heraeus Kulzer GmbH, 63450 Hanau (DE)
(72) Erfinder: Vogt, Sebastian, Dr., 99084 Erfurt (DE); Schnabelrauch, Matthias, Dr., 07749 Jena (DE); Kühn, Klaus-Dieter, Dr., 35041 Marburg (DE)
(74) Vertreter: Kühn, Hans-Christian

(56) Entgegenhaltungen:
- DE-A- 3 206 044
- US-A- 3 375 165

## Beschreibung

Die vorliegende Erfindung betrifft (interkonnektierend) poröse Körper mit antibiotischer Beschichtung, Verfahren zur Herstellung sowie ihre Verwendung. Diese antibiotisch ausgerüsteten porösen Körper sollen als Implantate in der Human- und Veterinärmedizin zur Behandlung von Knochendefekten und gegebenenfalls zur Behandlung von Weichteildefekten Verwendung finden. Dabei wird eine kontinuierliche Antibiotika-Freisetzung aus der auf der inneren Oberfläche der Porensysteme befindlichen antibiotischen Beschichtung über einen Zeitraum von mehreren Tagen angestrebt, damit eine bakterielle Infektion im Bereich des zu behandelnden Knochendefekts und/oder Weichteildefektes wirksam verhindert oder bekämpft werden kann. Insbesondere sollen solche bakteriellen Erreger behandelt werden, die Resistenzen gegenüber den üblich verwendeten Antibiotika ausgebildet haben.

Knochendefekte treten in der Human- und Veterinärmedizin relativ häufig auf und werden insbesondere durch Knochenfisteln, Trümmerfrakturen und Tumoren verursacht. Die Behandlung von Knochendefekten kann durch Auffüllung mit geeigneten Implantaten erfolgen. In den letzten Jahren haben insbesondere poröse Implantate Interesse gefunden, die auf Grund ihrer chemischen Zusammensetzung und ihrer Porosität eine osteokonduktive Wirkung aufweisen und ein Einwachsen des umgebenden Knochengewebes begünstigen. Problematisch ist die Behandlung von Knochendefekten immer dann, wenn zusätzlich bakterielle Infektionen des Knochengewebes vorhanden sind. Infektionen des Knochengewebes können nach vorheriger chirurgischer Sanierung durch systemische oder lokale Applikation von geeigneten Antibiotika bekämpft werden. Die systemische Anwendung von Antibiotika ist aufgrund der mitunter nicht unbeträchtlichen Toxizität der Antibiotika problematisch. Die lokale Applikation direkt im oder am infizierten Gewebe, nach entsprechender chirurgischer Sanierung, bietet dagegen den Vorteil, dass hohe lokale Antibiotika-Konzentrationen erreicht werden können unter Vermeidung von schädigenden Antibiotika-Konzentrationen im übrigen Organismus. Durch diese hohen lokalen Antibiotika-Konzentrationen am Ort der bakteriellen Infektion ist eine weitgehende Abtötung der Mikroorganismen möglich, so dass die bakteriellen Infektionen sehr wirksam behandelt werden. Besonders vorteilhaft ist es, wenn am Ort der bakteriellen Infektionen eine wirksame Antibiotikum-Konzentration über einen Zeitraum von mehreren Tagen bis Wochen aufrecht erhalten wird, damit das Antibiotikum möglichst tief in das infizierte Gewebe eindringen kann und dadurch auch schwer zugängliche Keime abgetötet werden. Weichteildefekte mit bakteriellen Infektionen sind in der Human- und Veterinärmedizin ebenfalls häufig zu finden. Die lokale Antibiotika-Applikation ist auch zur Behandlung dieser Infektionen von Interesse.

Bisher fanden schwerlösliche Salze der Aminoglykosid-Antibiotika und der Lincosamid-Antibiotika relativ geringe Beachtung für die Herstellung von Depotpräparaten und von antibiotisch wirksamen Implantaten. Bei den Aminoglykosid-Antibiotika sind einige wenige geringlösliche Salze bekannt. So wurde beim Gentamicin die Darstellung geringlöslicher Salze basierend auf höheren Fettsäuren, Arylalkylcarbonsäuren, Alkylsulfaten und Alkylsulfonaten beschrieben (G. M. Luedemann, M. J. Weinstein: Gentamycin and method of production. 16.07.1962, US 3,091,572). Exemplarisch sind dafür Gentamicin-Salze der Laurinsäure, der Stearinsäure, der Palmitinsäure, der Ölsäure, der Phenylbuttersäure, der Naphthalen-1-carbonsäure. Die Synthese der Dodecylsulfate des Gentamicins in wäßriger bzw. wäßrig-methanolischer Lösung sind von Jurado Soler et al. beschrieben worden (A. Jurado Soler, J. A. Ortiz Hernandez, C. Ciuro Bertran: Neue Gentamicinderivate, Verfahren zur Herstellung derselben und diese enthaltende antibiotisch wirksame Zusammensetzung. 30.09.1974, DE 24 46 640). Diese Salze erwiesen sich jedoch vielfach als unvorteilhaft, weil sie wachsartige, hydrophobe Substanzen darstellen, die eine galenische Verwendung behindern. Weiterhin wurden Fettsäuresalze und aliphatische Sulfate von Gentamicin und von Etamycin aus der freien Base bzw. aus ihren Salzen in Wasser bei 50-80°C synthetisiert (H. Voege, P. Stadler, H. J. Zeiler, S. Samaan, K. G. Metzger: Schwerlösliche Salze von Aminoglykosiden sowie diese enthaltende Formulierungen mit verzögerter Wirkstoff-Freigabe. 28.12.1982, DE 32 48 328). Diese Antibiotika-Fettsäuresalze sollten als Injektionspräparate geeignet sein. Eine neuere Entwicklung stellen schwerlösliche Aminoglykosid-Flavonoid-Phosphate dar (H. Wahlig, E. Dingeldein, R. Kirchlechner, D. Orth, W. Rogalski: Flavonoid phosphate salts of aminoglycoside antibiotics. 13.10.1986, US 4,617,293). Es werden die Salze der Phosphorsäuremonoester von Derivaten der Hydroxyflavane, Hydroxyflavene, Hydroxyflavanone, Hydroxyflavone und Hydroxyflavylium beschrieben. Besonders bevorzugt sind dabei die Derivate der Flavanone und der Flavone. Diese schwerlöslichen Salze sollen als Depotpräparate Verwendung finden. So werden z. B. diese Salze in Kollagenvliese eingebracht (H. Wahlig, E. Dingeldein, D. Braun: Medicinally useful, shaped mass of collagen resorbable in the body. 22.09.1981, US 4,291,013). Weiterhin wurden auch künstliche Herzklappen mit diesen schwerlöslichen Gentamicin-Salzen, Gentamicin Crobefat, imprägniert (M. Cimbollek, B.Nies, R. Wenz, J. Kreuter: Antibiotic-impregnated heart valve sewing rings for treatment and prophylaxis of bacterial endocarditis. Antimicrob. Agents Chemother. 40(6) (1996)1432-1437).

Die Erzeugung von einfachen Antibiotikum-/Antibiotika-Depots in den Porensystemen von porösen Körpern durch Tränken von porösen Körpern mit wäßrigen Antibiotika-Lösungen ist allgemeiner Kenntnisstand (R. Reiner, W. Kißing, H. Döring, K. Köster, H. Heide: Implantierbares Pharmaka-Depot. 20.02.1978, DE 28 07 132). Hierbei kann eine retardierende Wirkstofffreisetzung des in Wasser löslichen Wirkstoffs nur durch Adsorptions- und/oder durch Diffusionsprozesse erreicht werden, die vom verwendeten Material, dem Porenvolumen und der Porosität abhängt. Daneben ist es auch möglich, in Wasser gering lösliche Antibiotika-Salze in geeigneten organischen Lösungsmitteln zu lösen und mit diesen Lösungen die Formkörper zu tränken. Dadurch entstehen Wirkstoffdepots in den Formkörpern, die eine retardierende Wirkstofffreisetzung zeigen. Ein Beispiel dafür ist die von Cimbollek und Nies beschriebene Methode zur Lösung eines in Wasser gering löslichen Gentamicin-Salzes und deren Verwendung zur Beschichtung (M. Cimbollek, B. Nies: Solvent for a sparinly soluble gentamicin salt. 04.05.1994, US 5,679,646). Dieses Gentamicinsalz auf Basis von 3-p-Methoxybezylidene-6-hydroxy-4'methoxyflavanone-6-phosphat muss jedoch vor der Beschichtung synthetisiert werden. Von Kurtz wird eine interessante Variante beschrieben, bei der in Wasser gering lösliche Antibiotika-Salze in situ in einer nicht näher spezifizierten Unterlage durch aufeinanderfolgende Tränkung mit einer Lösung eines basischen Gentamicin-Salzes bzw. eines Polymycin-Salzes und eines sauren Penicillin- bzw. Cephalosporinsalzes gebildet werden (L. D. Kurtz: Wasserunlösliche biocide Antibiotikasalze. 13.11.1973, DE 23 01 633). Die Penicillin- bzw. Cephalosporinreste bilden die anionische Komponente der Salze und die kationischen Aminoglukosid-Reste die kationische Komponente.

Fusidinsäure ist ein Steroidantibiotikum, dass insbesondere für die Behandlung von Infektionen von Staphylokokken von Bedeutung ist. Bisher fand dieses Antibiotikum nur geringe Beachtung zur Herstellung von Implantaten. So wird in DE 32 06 044 A1 ein implantierbares pharmazeutisches Mittel und ein Verfahren zur Herstellung dieses Mittels beschrieben. Das Mittel enthält CaSO₄ mit ½ bis 2 mol H₂O und zumindest Fusidinsäure und/oder Gentamicin oder deren Salzen, gegebenenfalls in Kombination mit anderen bakteriellen Substanzen. Es wird ausgeführt, dass die antibiotische Substanz eine Mischung aus Fusidinsäure oder einem ihrer Salze und Gentamicin oder einem seiner Salze ist. In der Beschreibung wird vorgeschlagen, zusätzlich weitere Antibiotika einzubringen. In diesem Fall muss die Freisetzungsgeschwindigkeit jeder der einzelnen Komponenten berücksichtigt werden.

Bisher sind keine antibiotischen Beschichtungen in porösen Körpern unter Nutzung von geringlöslichen Antibiotika-Salzen der Fusidinsäure bekannt.

Der Erfindung liegt die Aufgabe zu Grunde, eine antibiotische Beschichtung von porösen Körpern zu entwickeln, die im wäßrigen Milieu Antibiotika verzögert über einen Zeitraum von mehreren Tagen bis zu wenigen Wochen kontinuierlich freisetzt.

Die Aufgabe wird erfindungsgemäß durch Merkmale der unabhängigen Ansprüche gelöst. Vorteilhafte Ausführungen sind in den Unteransprüchen angegeben.

Der Erfindung liegt der überraschende Befund zu Grunde, dass Fusidinsäure mit kationischen Antibiotika aus den Gruppen der Aminoglykosid-Antibiotika, der Lincosamid-Antibiotika, der Chinolon-Antibiotika, der Peptid-Antibiotika und der Tetracyclin-Antibiotika in Wasser gering lösliche Salze bildet und diese Antibiotika-Fusidinsäure-Salze Beschichtungen auf der Oberfläche von porösen Körpern ausbilden. Diese Beschichtungen setzen im wäßrigen Milieu über einen Zeitraum von mehreren Tagen bei 37°C kontinuierlich Antibiotika frei.

Diese schichtbildenden Salze sind durch Umsetzung von in Wasser löslichen Salzen der Fusidinsäure, wie z. B. dem Natriumsalz der Fusidinsäure, mit in Wasser löslichen Salzen des Gentamicins, des Sisomycins, des Netilmicins, des Streptomycins, des Tobramycins, des Spectinomycins, des Vancomycins, des Ciprofloxacins, des Moxifloxacins, des Clindamycins, Lincomycins, des Tetracyclins, des Chlortetracyclins, des Oxytetracyclins und des Rolitetracyclins zugänglich. Die Herstellung der Antibiotikum-Fusidinsäure-Salze ist ein reziproker Salzaustausch. Die anionische Komponente dieser Komplexe wird durch die Fusidinat-Anionen gebildet und die kationische Komponente wird durch die kationischen, protonierten Antibiotika-Basen des Gentamicins, des Sisomycins, des Netilmicins, des Streptomycins, des Tobramycins, des Spectinomycins, des Vancomycins, des Ciprofloxacins, des Moxifloxacins, des Clindamycins, des Lincomycins, des Tetracyclins, des Chlortetracyclins, des Oxytetracyclins und des Rolitetracyclins gebildet. Im folgenden werden diese Fusidinsäure-Antibiotika-Salze vereinfacht als Fusidinsäure-Gentamicin, Fusidinsäure-Sisomycin, Fusidinsäure-Netilmicin, Fusidinsäure-Streptomycin, Fusidinsäure-Tobramycin, Fusidinsäure-Spectinomycin, Fusidinsäure-Vancomycin, Fusidinsäure-Ciprofloxacin, Fusidinsäure-Moxifloxacin, Fusidinsäure-Clindamycin, Fusidinsäure-Lincomycin Fusidinsäure-Tetracyclin, Fusidinsäure-Chlortetracyclin, Fusidinsäure-Oxytetracyclin und Fusidinsäure-Rolitetracyclin bezeichnet. Diese Fusidinsäure-Antibiotika-Salze umfassen alle möglichen antibiotischen Salze mit einem Stoffmengenverhältnis von Fusidinsäure zu der protonierten Antibiotikabase von 1 zu 1 bis 1 zu 5.

Im Sinne der Erfindung ist zweckmäßig, dass die antibiotischen Beschichtung anstelle von Fusidinsäure-Anionen antibiotisch wirksame Anionen von Fusidinsäurederivaten enthält und dass antibiotisch wirksame Salze von Fusidinsäurederivaten anstelle von Salzen der Fusidinsäure zur Herstellung der erfindungsgemäßen antibiotischen Beschichtung verwendet werden.

Es ist vorteilhaft, dass eine Beschichtung aus mindestens einem in Wasser oder im wäßrigem Milieu gering löslichen Antibiotika-Salz aus der Gruppe des Fusidinsäure-Gentamicins, des Fusidinsäure-Sisomycins, des Fusidinsäure-Netilmicin, des Fusidinsäure-Streptomycins, des Fusidinsäure-Tobramycins, des Fusidinsäure-Spectinomycins, des Fusidinsäure-Vancomycins, des Fusidinsäure-Ciprofloxacins, des Fusidinsäure-Moxifloxacins, des Fusidinsäure-Clindamycins, des Fusidinsäure-Lincomycins, des Fusidinsäure-Tetracyclinss, des Fusidinsäure-Chlortetracyclins, des Fusidinsäure-Oxytetracyclins und des Fusidinsäure-Rolitetracyclins in das Porensystem von nichtmetallischen porösen Körpern und/oder von metallischen Körpern eingebracht ist.

Weiterhin ist erfindungsgemäß, dass zuerst eine wäßrige Lösung, die mindestens einen Vertreter eines in Wasser löslichen Salzes des Gentamicins, des Sisomycins, des Netilmicins, des Streptomycins, des Tobramycins, des Spectinomycins, des Vancomycins, des Ciprofloxacins, des Moxifloxacins, des Clindamycins, des Lincomycins, des Tetracyclins, des Chlortetracyclins, des Oxytetracyclins und des Rolitetracyclins enthält, in das Porensystem von porösen Körpern eingebracht wird und dass nach einem Trocknungsschritt eine zweite wäßrige Lösung eines in Wasser leicht löslichen Salzes der Fusidinsäure eingebracht wird und dabei eine in Wasser gering lösliche antibiotische Beschichtung im Porensystem des porösen Körpers ausgebildet wird.

Es kann vorteilhaft sein, dass die Reihenfolge der Beschichtungsschritte vertauscht wird.

Auch ist zweckmäßig, dass die antibiotische Beschichtung auf poröse Körper, die in Form von porösen Pulvern, porösen Granulaten, porösen Formkörpern und/oder porösen Schichten auf kompakten Körpern vorliegen, aufgebracht ist.

Es ist vorteilhaft, dass die antibiotische Beschichtung von porösen Körpern, die vorzugsweise in Form von porösen Pulvern und/oder Granulaten vorliegen, durch Zusatz von mindestens einem in Wasser oder im wäßrigem Milieu gering löslichen Antibiotika-Salz aus der Gruppe Fusidinsäure-Gentamicin, Fusidinsäure-Sisomycin, Fusidinsäure-Netilmicin, Fusidinsäure-Streptomycin, Fusidinsäure-Tobramycin, Fusidinsäure-Spectinomycin, Fusidinsäure-Vancomycin, Fusidinsäure-Ciprofloxacin, Fusidinsäure-Moxifloxacin, Fusidinsäure-Clindamycin, Fusidinsäure-Lincomycin, Fusidinsäure-Tetracyclin, Fusidinsäure-Chlortetracyclin, Fusidinsäure-Oxytetracyclin und Fusidinsäure-Rolitetracyclin insbesondere durch Vermahlen, unter Zusatz von Methanol, Ethanol, Dioxan, Tetrahydrofuran, Dimethylsulfoxid und/oder Wasser oder Gemische gebildet ist.

Ebenfalls ist es vorteilhaft, dass die antibiotische Beschichtung von porösen Körpern, die vorzugsweise in Form von porösen Pulvern und/oder Granulaten aufweisen, durch Zusatz von einem Gemisch aus mindestens einem in Wasser löslichen Salzes des Gentamicins, des Sisomycins, des Netilmicins, des Streptomycins, des Tobramycins, des Spectinomycins, des Vancomycins, des Ciprofloxacins, des Moxifloxacins, des Clindamycins, des Lincomycins, des Tetracyclins, des Chlortetracyclins, des Oxytetracyclins und des Rolitetracyclins und mindestens einem in Wasser löslichen Salzes der Fusidinsäure in Gegenwart von Wasser oder wäßrigen Lösungen, insbesondere durch Vermahlen, gebildet ist.

Es ist zweckmäßig, dass die antibiotische Beschichtung gegebenenfalls zusätzlich in Wasser lösliche Salze des Gentamicins, des Sisomycins, des Netilmicins, des Streptomycins, des Tobramycins, des Spectinomycins, des Vancomycins, des Ciprofloxacins, des Moxifloxacins, des Clindamycins, des Lincomycins, des Tetracyclins, des Chlortetracyclins, des Oxytetracyclins und des Rolitetracyclins enthält.

Zweckmäßig ist weiterhin, dass die antibiotische Beschichtung auf resorbierbare, poröse Körper, auf partiell resorbierbare poröse Körper und/oder auf nicht resorbierbare, biokompatible poröse Körper aufgebracht ist.

Erfindungsgemäß ist die Verwendung von porösen Körpern mit einer antibiotischen Beschichtung, die in Form von beschichteten porösen Granulaten und/oder beschichteten porösen Pulvern zu Formkörpern verpresst sind, als/für Implantate.

Es ist erfindungsgemäß, dass die antibiotisch beschichteten poröse Granulate und/oder antibiotisch beschichteten poröse Pulver als Bindemittel für die Herstellung von Formkörpern durch Verpressung von Pulvergemischen verwendet werden.

Es ist erfindungsgemäß, dass die porösen Körper mit antibiotischer Beschichtung für temporäre oder dauerhafte Implantate verwendet werden.

Wesentlich für die Erfindung ist die Verwendung von porösen Körpern mit einer erfindungsgemäßen antibiotischen Beschichtung als Antibiotika-Depot von Implantaten.

Die Erfindung soll nachfolgend durch die Beispiele 1 - 8 näher erläutert werden, ohne die Erfindung jedoch einzuschränken.

### Beispiel 1

Es werden 400,0 mg poröses Calciumsulfat-Dihydrat durch Vermahlen mit einem Gemisch aus 100,0 mg Poly-L-lactid (M ∼ 10000 g/mol) und 20,0 mg Gentamicin-Fusidinsäure beschichtet. Jeweils 200 mg des beschichteten Calciumsulfat-Dihydrates werden mit einer Presse bei einem Druck von 5 Tonnen innerhalb von zwei Minuten zu scheibenförmigen Formkörpern, mit einem Durchmesser von 13 mm, gepresst.

### Beispiel 2

Es werden 400,0 mg Calciumsulfat-Dihydrat durch Vermahlen mit einem Gemisch aus 100,0 mg Poly-L-lactid (M ∼ 10000 g/mol) und 20,0 mg Lincomycin-Fusidinsäure beschichtet. Jeweils 200 mg des so beschichteten Calciumsulfat-Dihydrates werden mit einer Presse bei einem Druck von 5 Tonnen innerhalb von zwei Minuten zu scheibenförmigen Formkörpern, mit einem Durchmesser von 13 mm, gepresst.

### Beispiel 3

Es werden 400,0 mg poröses Calciumsulfat-Dihydrat durch Vermahlen mit einem Gemisch aus 100,0 mg Poly-L-lactid (M ∼ 10000 g/mol) und 20,0 mg Sisomycin-Fusidinsäure beschichtet. Jeweils 200 mg des beschichteten Calciumsulfat-Dihydrates werden mit einer Presse bei einem Druck von 5 Tonnen innerhalb von zwei Minuten zu scheibenförmigen Formkörpern, mit einem Durchmesser von 13 mm, gepresst.

### Beispiel 4

Es werden 400,0 mg poröses Calciumsulfat-Dihydrat durch Vermahlen mit einem Gemisch aus 100,0 mg Poly-L-lactid (M ∼ 10000 g/mol) und 20,0 mg Clindamycin-Fusidinsäure beschichtet. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepresst.

### Beispiel 5

Es werden 400,0 mg poröses Calciumsulfat-Dihydrat durch Vermahlen mit einem Gemisch aus 100,0 mg Poly-L-lactid (M ∼ 10000 g/mol) und 20,0 mg Tetracyclin-Fusidinsäure beschichtet. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepresst.

### Beispiel 6

Ein poröser Glaswürfel (Masse 3,8814 g, Porosität ∼ 60 %) wird zuerst mit 2,0 ml einer 0,5 Ma%igen wäßrigen Clindamycinhydrochlorid-Lösung getränkt und anschließend bis zur Massekonstanz bei 60 °C getrocknet. Die Masse des beschichteten Glaswürfels betrug nach der Trocknung 3,8909 g. Anschließend wurde der beschichtete Glaswürfel mit 2,0 ml einer 0,5 Ma%igen Fusidinsäure-Natriumsalz-Lösung nochmals getränkt und danach bis zur Massekonstanz getrocknet. Der getrocknete beschichtete Glaswürfel hatte eine Masse von 3,9011 g. Es hatte sich eine Beschichtung aus Clindamycin-Fusidinsäure gebildet, die auf der Oberfläche der porösen Glaswürfel haftete.

### Beispiel 7

Ein poröser Glaswürfel (Masse 3,9176 g, Porosität ∼ 60 %) wird zuerst mit 2,0 ml einer 0,5 Ma%igen wäßrigen Tetracyclinhydrochlorid-Lösung getränkt und anschließend bis zur Massekonstanz bei 60 °C getrocknet. Die Masse des beschichteten Glaswürfels betrug nach der Trocknung 3,9281 g. Anschließend wurde der beschichtete Glaswürfel mit 2,0 ml einer 0,5 Ma%igen Fusidinsäure-Natriumsalz-Lösung nochmals getränkt und danach bis zur Massekonstanz getrocknet. Es hatte sich eine Beschichtung aus Tetracyclin-Fusidinsäure gebildet, die auf der Oberfläche der porösen Glaswürfel haftete. Der getrocknete beschichtete Glaswürfel hatte eine Masse von 3,9384 g.

### Beispiel 8

Ein poröser Glaswürfel (Masse 4,0953 g, Porosität ∼ 60 %) wird zuerst mit 2,0 ml einer 0,5 Ma%igen wäßrigen Gentamicinsulfat-Lösung getränkt und anschließend bis zur Massekonstanz bei 60 °C getrocknet. Die Masse des beschichteten Glaswürfels betrug nach der Trocknung 4,1038 g. Anschließend wurde der beschichtete Glaswürfel mit 2,0 ml einer 0,5 Ma%igen Fusidinsäure-Natriumsalz-Lösung nochmals getränkt und danach bis zur Massekonstanz getrocknet. Der getrocknete beschichtete Glaswürfel hatte eine Masse von 4,1150 g.

### Antibiotika-Freisetzungsversuche

Die in den Beispielen 1-5 hergestellten Formkörper und die in den Beispielen 6-8 beschichteten porösen Glaskörper wurden in Sörensen-Puffer pH 7,4 eingebracht und in diesem bei 37 °C über einen Zeitraum von 7 Tagen gelagert. Bei den Beispielen 1-5 wurden nach 7 Tagen die Freisetzungsversuche abgebrochen und bei den Beispielen 6-8 nach 8 Tagen. Die Probennahme erfolgte täglich, wobei das Freisetzungsmedium ausgetauscht wurde. Die Antibiotika-Freisetzung aus den Formkörpern wurde mit einem Agardiffusionstest unter Verwendung von Bacillus subtilis ATCC 6633 als Testkeim verfolgt. Es wurden die Hemmhofdurchmesser mit Hilfe eines Scanners und einer speziellen Auswertungssoftware ermittelt. Diem Egebnisse der Freisetzungsversuche sind in den Tabellen 1 - 3 dargestellt.

**Tab.1**

| Zeit [d] | Beispiel 1 | | Beispiel 2 | |
|---|---|---|---|---|
| | Verdünnung | Hemmhofdurchmesser [mm] | Verdünnung | Hemmhofdurchmesser [mm] |
| 1 | 1:50 | 17,90 | unverdünnt | 22,60 |
| 2 | unverdünnt | 25,50 | unverdünnt | 19,10 |
| 3 | unverdünnt | 26,35 | unverdünnt | 17,45 |
| 4 | unverdünnt | 24,80 | unverdünnt | 13,30 |
| 5 | unverdünnt | 22,45 | unverdünnt | 15,40 |
| 6 | unverdünnt | 19,45 | unverdünnt | 12,40 |
| 7 | unverdünnt | 16,50 | unverdünnt | 12,55 |

**Tab.2**

| Zeit [d] | Beispiel 3 | | Beispiel 4 | | Beispiel 5 | |
|---|---|---|---|---|---|---|
| | Verdünnung | Hemmhofdurchmesser [mm] | Verdünnung | Hemmhofdurchmesser [mm] | Verdünnung | Hemmhofdurchmesser [mm] |
| 1 | 1: 50 | 16,90 | 1:100 | 18,90 | 1:10 | 19,50 |
| 2 | unverdünnt | 24,70 | unverdünnt | 22,50 | unverdünnt | 21,73 |
| 3 | unverdünnt | 26,20 | unverdünnt | 20,85 | unverdünnt | 21,48 |
| 4 | unverdünnt | 24,40 | unverdünnt | 19,30 | unverdünnt | 19,25 |
| 5 | unverdünnt | 25,10 | unverdünnt | 20,00 | unverdünnt | 21,15 |
| 6 | unverdünnt | 21,90 | unverdünnt | 17,30 | unverdünnt | 19,00 |
| 7 | unverdünnt | 18,50 | unverdünnt | 17,00 | unverdünnt | 17,50 |

**Tab.3**

| Zeit [d] | Beispiel 6 | | Beispiel 7 | | Beispiel 8 | |
|---|---|---|---|---|---|---|
| | Verdünnung | Hemmhofdurchmesser [mm] | Verdünnung | Hemmhofdurchmesser [mm] | Verdünnung | Hemmhofdurchmesser [mm] |
| 1 | 1:20 | 23,15 | 1:50 | 15,13 | 1:50 | 22,10 |
| 2 | 1:10 | 19,25 | 1:10 | 16,85 | 1:10 | 22,53 |
| 3 | 1:2 | 19,58 | 1:5 | 17,03 | 1:5 | 21,58 |
| 4 | unverdünnt | 18,40 | 1:2 | 18,48 | 1:2 | 21,58 |
| 5 | unverdünnt | 14,10 | unverdünnt | 21,73 | unverdünnt | 21,50 |
| 6 | unverdünnt | 11,40 | unverdünnt | 20,03 | unverdünnt | 19,70 |
| 7 | unverdünnt | 0,00 | unverdünnt | 20,53 | unverdünnt | 18,75 |
| 8 | unverdünnt | 0,00 | unverdünnt | 19,43 | unverdünnt | 17,55 |

## Patentansprüche

1. Poröser Körper mit antibiotischer Beschichtung, **dadurch gekennzeichnet, dass** eine Beschichtung aus mindestens einem in Wasser oder im wäßrigem Milieu gering lösliches Antibiotika-Salz aus der Gruppe des Fusidinsäure-Gentamicins, des Fusidinsäure-Sisomycins, des Fusidinsäure-Netilmicin, des Fusidinsäure-Streptomycins, des Fusidinsäure-Tobramycins, des Fusidinsäure-Spectinomycins, des Fusidinsäure-Vancomycins, des Fusidinsäure-Ciprofloxacins, des Fusidinsäure-Moxifloxacins, des Fusidinsäure-Clindamycins, des Fusidinsäure-Lincomycins, des Fusidinsäure-Tetracyclinss, des Fusidinsäure-Chlortetracyclins, des Fusidinsäure-Oxytetracyclins und des Fusidinsäure-Rolitetracyclins in das Porensystem von nichtmetallischen porösen Körpern und/oder von metallischen Körpern eingebracht ist.

2. Poröser Körper nach Anspruch 1 in Form von porösen Pulvern, porösen Granulaten, porösen Formkörpern und/oder porösen Schichten auf kompakten Körpern.

3. Poröser Körper nach einem der vorstehenden Ansprüche, der biokompatibel und resorbierbar, partiell resorbierbar, oder nicht resorbierbar ist.

4. Poröser Körper nach nach einem der vorstehenden Ansprüche , **dadurch gekennzeichnet, dass** die antibiotische Beschichtung durch Zusatz von mindestens einem in Wasser oder im wäßrigem Milieu gering löslichen Antibiotika-Salz aus der Gruppe des Fusidinsäure-Gentamicins, des Fusidinsäure-Sisomycins, des Fusidinsäure-Netilmicins, des Fusidinsäure-Streptomycins, des Fusidinsäure-Tobramycins, des Fusidinsäure-Spectinomycins, des Fusidinsäure-Vancomycins, des Fusidinsäure-Ciprofloxacins, des Fusidinsäure-Moxifloxacins, des Fusidinsäure-Clindamycins, des Fusidinsäure-Lincomycins, des Fusidinsäure-Tetracyclins, des Fusidinsäure-Chlortetracyclins, des Fusidinsäure-Oxytetracyclins und des Fusidinsäure-Rolitetracyclins insbesondere durch Vermahlen, unter Zusatz von Methanol, Ethanol, Dioxan, Tetrahydrofuran, Dimethylsulfoxid und/oder Wasser oder Gemische gebildet ist.

5. Poröser Körper nach einem der vorstehenden Ansprüche, **dadurch gekennzeichnet, dass** die antibiotische Beschichtung durch Zusatz von einem Gemisch aus mindestens einem in Wasser löslichen Salzes des Gentamicins, des Sisomycins, des Netilmicins, des Streptomycins, des Tobramycins, des Spectinomycins, des Vancomycins, des Ciprofloxacins, des Moxifloxacins, des Clindamycins, des Lincomycins, des Tetracyclins, des Chlortetracyclins, des Oxytetracyclins und des Rolitetracyclins und mindestens einem in Wasser löslichen Salzes der Fusidinsäure in Gegenwart von Wasser oder wäßrigen Lösungen, insbesondere durch Vermahlen, gebildet ist.

6. Poröser Körper nach einem der vorstehenden Ansprüche, **dadurch gekennzeichnet, dass** die antibiotische Beschichtung gegebenenfalls zusätzlich in Wasser lösliche Salze des Gentamicins, des Sisomycins, des Netilmicins, des Streptomycins, des Tobramycins, des Spectinomycins, des Vancomycins, des Ciprofloxacins, des Moxifloxacins, des Clindamycins, des Lincomycins, des Tetracyclins, des Chlortetracyclins, des Oxytetracyclins und des Rolitetracyclins enthält.

7. Verfahren zur Herstellung von porösen Körpern nach einem der Ansprüche 1 bis 6, **dadurch gekennzeichnet, dass** zuerst eine wäßrige Lösung, die mindestens einen Vertreter eines in Wasser löslichen Salzes des Gentamicins, des Sisomycins, des Netilmicins, des Streptomycins, des Tobramycins, des Spectinomycins, des Vancomycins, des Ciprofloxacins, des Moxifloxacins, des Clindamycins, des Lincomycins, des Tetracyclins, des Chlortetracyclins, des Oxytetracyclins und des Rolitetracyclins enthält, in das Porensystem von porösen Körpern eingebracht wird und dass nach einem Trocknungsschritt eine zweite wäßrige Lösung eines in Wasser leicht löslichen Salzes der Fusidinsäure eingebracht wird und dabei eine in Wasser gering lösliche antibiotische Beschichtung im Porensystem des porösen Körpers ausgebildet wird.

8. Verfahren nach Anspruch 7, **dadurch gekennzeichnet, dass** die Reihenfolge der Beschichtungsschritte vertauscht wird.

9. Verwendung von porösen Körpern nach einem der Ansprüche 1 bis 6, die in Form von beschichteten porösen Granulaten und/oder beschichteten porösen Pulvern zu Formkörpern verpresst sind, zur Herstellung von Implantaten.

10. Verwendung von porösen Körpern nach einem der Ansprüche 1 bis 6, die als antibiotisch beschichtete poröse Granulate und/oder antibiotisch beschichtete poröse Pulver ausgebildet sind, als Bindemittel für die Herstellung von Formkörpern durch Verpressung von Pulvergemischen.

11. Verwendung von porösen Körpern nach einem der Ansprüche 1 bis 6, zur Herstellung temporärer oder dauerhafter Implantate.

12. Verwendung von porösen Körpern nach einem der Ansprüche 1 bis 6 als Antibiotika-Depot bei der Herstellung von Implantaten.

## Claims

1. A porous body comprising an antibiotic coating, **characterised in that** a coating of at least an antibiotic salt, which is sparingly soluble in water or in an aqueous medium, from the group of fusidic acid-gentamicin, fusidic acid-sisomicin, fusidic acid-netilmicin, fusidic acid-streptomycin, fusidic acid-tobramycin, fusidic acid-spectinomycin, fusidic acid-vancomycin, fusidic acid-ciprofloxacin, fusidic acid-moxifloxacin, fusidic acid-clindamycin, fusidic acid-lincomycin, fusidic acid-tetracycline, fusidic acid-chlorotetracycline, fusidic acid-oxytetracycline and fusidic acid-rolitetracycline is introduced into the pore system of non-metallic porous bodies and/or metallic bodies.

2. Porous body according to claim 1 in the form of porous powders, porous granulates, porous moulded bodies and/or porous layers on solid bodies.

3. Porous body according to any one of the preceding claims which is biocompatible and resorbable, partially resorbable, or non-resorbable.

4. Porous body according to any one of the preceding claims, **characterised in that** the antibiotic coating is formed by adding, in particular by grinding, at least an antibiotic salt, which is sparingly soluble in water or in an aqueous medium, from the group of fusidic acid-gentamicin, fusidic acid-sisomicin, fusidic acid-netilmicin, fusidic acid-streptomycin, fusidic acid-tobramycin, fusidic acid-spectinomycin, fusidic acid-vancomycin, fusidic acid-ciprofloxacin, fusidic acid-moxifloxacin, fusidic acid-clindamycin, fusidic acid-lincomycin, fusidic acid-tetracycline, fusidic acid-chlorotetracycline, fusidic acid-oxytetracycline and fusidic acid-rolitetracycline and with the addition of methanol, ethanol, dioxane, tetrahydrofuran, dimethyl sulfoxide and/or water, or mixtures thereof.

5. Porous body according to any one of the preceding claims, **characterised in that** the antibiotic coating is formed by adding, in particular by grinding, a mixture of at least a water-soluble salt of gentamicin, sisomicin, netilmicin, streptomycin, tobramycin, spectinomycin, vancomycin, ciprofloxacin, moxifloxacin, clindamycin, lincomycin, tetracycline, chlorotetracycline, oxytetracycline and rolitetracycline, and at least a water-soluble salt of fusidic acid in the presence of water or aqueous solutions.

6. Porous body according to any one of the preceding claims, **characterised in that** the antibiotic coating optionally additionally contains water-soluble salts of gentamicin, sisomicin, netilmicin, streptomycin, tobramycin, spectinomycin, vancomycin, ciprofloxacin, moxifloxacin, clindamycin, lincomycin, tetracycline, chlorotetracycline, oxytetracycline, and rolitetracycline.

7. Method for producing porous bodies according to any one of claims 1 to 6, **characterised in that** an aqueous solution containing at least one representative of a water-soluble salt of gentamicin, sisomicin, netilmicin, streptomycin, tobramycin, spectinomycin, vancomycin, ciprofloxacin, moxifloxacin, clindamycin, lincomycin, tetracycline, chlorotetracycline, oxytetracycline and rolitetracycline is first introduced into the pore system of porous bodies, and **in that** following a drying step a second aqueous solution of a readily water-soluble salt of fusidic acid is introduced, thereby forming a sparingly water-soluble antibiotic coating in the pore system of the porous body.

8. Method according to claim 7, **characterised in that** the order of the coating steps is changed.

9. Use of porous bodies according to any one of claims 1 to 6 for producing implants, which porous bodies in the form of coated porous granulates and/or coated porous powders are compressed to create moulded bodies.

10. Use of porous bodies according to any one of claims 1 to 6 as binders for producing moulded bodies by compressing powder compounds, which porous bodies are formed as antibiotically-coated porous granulates and/or antibiotically-coated porous powders.

11. Use of porous bodies according to any one of claims 1 to 6 for producing temporary or permanent implants.

12. Use of porous bodies according to any one of claims 1 to 6 as a repository for antibiotics when producing implants.

## Revendications

1. Corps poreux ayant un revêtement antibiotique, **caractérisé en ce qu'**un revêtement obtenu à partir d'au moins un sel d'antibiotique peu soluble dans l'eau ou en milieu aqueux, du groupe de l'acide fusidique-gentamycine, de l'acide fusidique-sisomycine, de l'acide fusidique-nétilmicine, de l'acide fusidique-streptomycine, de l'acide fusidique-tobramycine, de l'acide fusidique-spectinomycine, de l'acide fusidique-vancomycine, de l'acide fusidique-ciprofloxacine, de l'acide fusidique-moxifloxacine, de l'acide fusidique-clindamycine, de l'acide fusidique-lincomycine, de l'acide fusidique-tétracycline, de l'acide fusidique-chlorotétracycline, de l'acide fusidique-oxytétracycline et de l'acide fusidique-rolitétracycline est introduit dans le système de pores de corps poreux non métalliques et/ou de corps métalliques.

2. Corps poreux selon la revendication 1 sous forme de poudres poreuses, de granulés poreux, de corps moulés poreux et/ou de couches poreuses sur des corps compacts.

3. Corps poreux selon l'une des revendications précédentes, qui est biocompatible et résorbable, partiellement résorbable ou non résorbable.

4. Corps poreux selon l'une des revendications précédentes, **caractérisé en ce que** le revêtement antibiotique est formé par addition d'au moins un sel d'antibiotique peu soluble dans l'eau ou en milieu aqueux, du groupe de l'acide fusidique-gentamycine, de l'acide fusidique-sisomycine, de l'acide fusidique-nétilmicine, de l'acide fusidique-streptomycine, de l'acide fusidique-tobramycine, de l'acide fusidique-spectinomycine, de l'acide fusidique-vancomycine, de l'acide fusidique-ciprofloxacine, de l'acide fusidique-moxifloxacine, de l'acide fusidique-clindamycine, de l'acide fusidique-lincomycine, de l'acide fusidique-tétracycline, de l'acide fusidique-chlorotétracycline, de l'acide fusidique-oxytétracycline et de l'acide fusidique-rolitétracycline, en particulier par broyage, avec addition de méthanol, d'éthanol, de dioxane, de tétrahydrofurane, de diméthylsulfoxyde et/ou d'eau ou de mélanges.

5. Corps poreux selon l'une des revendications précédentes, **caractérisé en ce que** le revêtement antibiotique est formé par addition d'un mélange d'au moins un sel soluble dans l'eau de la gentamycine, de la sisomycine, de la nétilmicine, de la streptomycine, de la tobramycine, de la spectinomycine, de la vancomycine, de la ciprofloxacine, de la moxifloxacine, de la clindamycine, de la lincomycine, de la tétracycline, de la chlorotétracycline, de l'oxytétracycline et de la rolitétracycline et d'au moins un sel soluble dans l'eau de l'acide fusidique en présence d'eau ou de solutions aqueuses, en particulier par broyage.

6. Corps poreux selon l'une des revendications précédentes, **caractérisé en ce que** le revêtement antibiotique contient éventuellement en outre des sels solubles dans l'eau de la gentamycine, de la sisomycine, de la nétilmicine, de la streptomycine, de la tobramycine, de la spectinomycine, de la vancomycine, de la ciprofloxacine, de la moxifloxacine, de la clindamycine, de la lincomycine, de la tétracycline, de la chlorotétracycline, de l'oxytétracycline et de la rolitétracycline.

7. Procédé de préparation de corps poreux selon l'une des revendications 1 à 6, **caractérisé en ce que** l'on introduit d'abord une solution aqueuse, qui contient au moins un représentant d'un sel soluble dans l'eau de la gentamycine, de la sisomycine, de la nétilmicine, de la streptomycine, de la tobramycine, de la spectinomycine, de la vancomycine, de la ciprofloxacine, de la moxifloxacine, de la clindamycine, de la lincomycine, de la tétracycline, de la chlorotétracycline, de l'oxytétracycline et de la rolitétracycline, dans le système de pores de corps poreux, et **en ce que**, après une étape de séchage, on introduit une deuxième solution aqueuse d'un sel bien soluble dans l'eau de l'acide fusidique, et on forme ainsi un revêtement antibiotique peu soluble dans l'eau dans le système de pores du corps poreux.

8. Procédé selon la revendication 7, **caractérisé en ce que** l'on échange l'ordre des étapes du revêtement.

9. Utilisation de corps poreux selon l'une des revendications 1 à 6 qui, sous forme de granulés poreux revêtus et/ou de poudres poreuses revêtues, sont comprimés en corps moulés, pour la préparation d'implants.

10. Utilisation de corps poreux selon l'une des revendications 1 à 6, qui sont sous forme de granulés poreux à revêtement antibiotique et/ou de poudres poreuses à revêtement antibiotique, comme liants pour la préparation de corps moulés par compression de mélanges de poudres.

11. Utilisation de corps poreux selon l'une des revendications 1 à 6 pour la préparation d'implants temporaires ou permanents.

12. Utilisation de corps poreux selon l'une des revendications 1 à 6 comme réserves d'antibiotiques dans la préparation d'implants.
